# EUROPÄISCHE PATENTSCHRIFT

(11) **EP 2 106 765 B1**
(45) Veröffentlichungstag und Bekanntmachung des Hinweises auf die Patenterteilung: **03.04.2013**
(21) Anmeldenummer: 08154024.7
(22) Anmeldetag: 03.04.2008
(51) Int. Cl.: A61B 19/00

(54) **Bildliche Orientierungshilfe für medizinische Instrumente**
Pictorial orientation aid for medical instruments
Aide à l'orientation imagée pour instruments médicaux

(43) Veröffentlichungstag der Anmeldung: 07.10.2009
(73) Patentinhaber: BrainLAB AG, 85622 Feldkirchen (DE)
(72) Erfinder: Christian, Georg, 81545 München (DE); Maier, Christian, 80802 München (DE); Lechner, Christian, 82287, Jesenwang (DE)
(74) Vertreter: Schwabe - Sandmair - Marx

(56) Entgegenhaltungen:
- EP-A- 1 002 502
- EP-A- 1 491 150
- WO-A-00/64367
- WO-A-02/09611
- US-A1- 2001 034 530
- US-A1- 2005 267 360

## Beschreibung

Die Erfindung betrifft eine bildliche Orientierungshilfe für medizinische Instrumente oder, anders ausgedrückt, ein Verfahren zur bildlichen Unterstützung der Ausrichtung eines medizinischen Instruments.

Die medizintechnische Navigation gestattet es, mit Hilfe eines Tracking- und Navigationssystems medizinische Instrumente zu orten und positionell zu verfolgen (Tracking). Die gezeigten Instrumente können dann auf einer Bildausgabe (zum Beispiel Bildschirm) im richtigen oder aktuellen positionellen Verhältnis zu einem Patientenkörper oder dessen Körperteilen dargestellt werden, wenn der Patient vorher mittels eines bildgebenden Verfahrens (CT, MR, etc.) gescannt und im Navigationssystem registriert wurde. In diesem Umfeld soll die vorliegende Erfindung eine bildliche Unterstützung bei der Ausrichtung bzw. Orientierung von Instrumenten geben.

Es ist oftmals notwendig, Instrumente genau auszurichten oder ihre Ausrichtung genau zu planen, zum Beispiel wenn Implantate genau an bestimmte Knochenstruktur-Positionen gesetzt werden müssen. Die Position eines Implantats wird dann beispielsweise durch einen Chirurgen eingestellt, der dabei benachbarte anatomische Landmarken, einzigartige Knochenstrukturen oder vorgeplante Punkte und Achsen in Betracht zieht. Beispielsweise muss bei der Fixierung einer Beckenfraktur die Trajektorie der eingesetzten Schraube einen speziellen Winkel zur seitlichen proximalen Oberschenkelknochen-Fläche haben, nämlich gewöhnlich etwa 130°. Um eine geeignete Stellung für die Beckenschraube einzustellen, wird gewöhnlich eine spezielle Bohrerführung verwendet, die an dem seitlichen Kortex des großen Trochanterbereichs angepresst wird. Das Führungsloch der Bohrerführung ist so ausgerichtet, dass seine Achse in einem Winkel von 130° zur proximalen Oberschenkelknochenachse auf den Oberschenkelhals zeigt.

Bei diesem Beispiel, aber auch bei anderen Eingriffen wird also ein separates und speziell gefertigtes Werkzeug benötigt, um ein medizinisches Instrument richtig einsetzen zu können. Außerdem benötigt dieses spezielle Werkzeug oftmals Kontakt mit normalerweise nicht freiliegenden Knochenstrukturen oder anderen Körperstrukturen, so dass oft eine weitgehende und invasive Vorbereitung, das heißt Öffnung des Patienten notwendig wird.

Das gleiche Problem tritt beispielsweise auch auf, wenn der Eintrittspunkt für spezielle Implantate vorbereitet werden muss, wie zum Beispiel ein lateraler Oberschenkelnagel, wobei ein spezieller Punkt am lateralen, proximalen Femur gefunden werden muss, indem man eine Schablone ansetzt. Auch hier sind stark invasive Vorbereitungen im Knochenbereich notwendig.

WO 02/09611 offenbart eine graphische Navigationshilfe für ein chirurgisches Instrument z.B. einen Bohrer. Diese Navigationshilfe umfasst ein Fadenkreuz, welches die Spitze des dargestellten Bohrers markiert. Eine tunnelartige Struktur und weitere Darstellungen auf einer Projektionsebene dienen als Anhaltspunkte zur Ausrichtung des Bohrers.

Es ist die Aufgabe der vorliegenden Erfindung, ein Verfahren zur bildlichen Unterstützung der Ausrichtung eines medizinischen Instruments bereitzustellen, welches den Einsatz spezieller Ausrichtungs-Werkzeuge unnötig macht. Insbesondere soll auch die Invasivität von Vorbereitungen zur Ausrichtung von Instrumenten möglichst stark verringert werden; speziell sollen invasive Techniken nur zur Instrumentenausrichtung möglichst vollständig wegfallen können.

Diese Aufgabe wird erfindungsgemäß durch ein Verfahren gemäß dem Anspruch 1 gelöst. Die Unteransprüche definieren bevorzugte Ausführungsformen der Erfindung.

Mit anderen Worten umfasst die Erfindung die Darstellung eines Kegels relativ zu einem speziellen Instrumententeil, und zwar auf einem Bilddatensatz, der mit einem medizintechnischen Navigationssystem verwendet wird. Es wird also eine Orientierungshilfe, die sowohl einen Bezug zum verwendeten Instrument als auch einen Bezug zum behandelten Patienten körperteil oder dessen Umgebung hat, in das Bilddatenmaterial mit der Abbildung des Instruments zusammen eingeblendet, und anhand dieser Einblendung kann dann von dem Verwender beurteilt werden, ob das Instrument richtig oder falsch ausgerichtet ist. Eine Hardware, also ein separates Ausrichtungswerkzeug, wird nicht mehr benötigt und kann dementsprechend eingespart werden. Ferner werden invasive Maßnahmen zum Ansetzen dieser Hardware (beispielsweise an einem Knochen) überflüssig und müssen nicht mehr durchgeführt werden.

Es ist möglich, die Musterform durch Einstellungen am Navigationssystem zu verändern, anzupassen oder auszutauschen bzw. eine Auswahl aus einer Anzahl von Musterformen zu treffen, wenn die Erfindung gemäß einer bevorzugten Ausführungsform umgesetzt wird.

In spezieller Ausgestaltung des erfindungsgemäßen Verfahrens unterstützt die Musterform eine Winkelorientierung des Instruments zum bzw. gegenüber dem

Die Musterform kann als Projektion in die Bildausgabe eingefügt werden, und hierbei ist es von Vorteil, wenn die Musterform angepasst auf die Bildebene der Patientenkörperteil-Darstellung projiziert und in der Projektion so bildlich dargestellt wird. Ein Beispiel: Ein bestimmter Winkel wird sich je nach Ausrichtung des Instruments vergrößern oder verkleinern, wenn das Instrument in verschiedenen Stellungen zur Bildebene gehalten wird. Es kommt deshalb darauf an, ob die Abbildung des virtuellen Winkels auf der Bildebene der Patientendaten übereinstimmt, und deshalb ist es von Vorteil, die Musterform je nach Lage auf diese Bildebene zu projizieren.

Gemäß einem weiteren Aspekt der Erfindung betrifft diese noch ein Verfahren zur Planung einer optimierten Instrumentenausrichtung mit Hilfe eines Bildunterstützungsverfahrens, wie es hierin in den unterschiedlichsten Ausführungsvarianten beschrieben wird.

Die Kegelform definiert einen bestimmten Öffnungswinkel an der Spitze der Abbildung eines chirurgischen Instruments auf einem Bilddatensatz, der in einem medizintechnischen Navigationssystem verwendet wird. Der Öffnungswinkel des Kegels kann in der Navigationssoftware-Anwendung eingestellt werden, und der Kegel soll als visuelle Hilfe dienen, um seine Umfangslinie mit einer gegebenen Achse auszurichten, wodurch ein halber Öffnungswinkel zwischen der Instrumentenachse und der vorgegebenen Achse erzielt wird. Der Kegelöffnungswinkel kann frei eingestellt werden, so dass die Erfindung für unterschiedliche Indikationen Verwendung finden kann. Auch erlaubt die Erfindung die Planung der Implantatpositionierung ohne physischen Kontakt irgendeiner Schablone mit der Knochenoberfläche. In derselben Weise könnte jedwede Form am Instrument oder an der Instrumentenspitze einprojiziert werden, um die genaue Planung der Implantatposition zu unterstützen, ohne mit irgendwelchen Schablonen invasiv auf den Knochen vorzudringen.

Die Erfindung kann beispielsweise immer dort verwendet werden, wo eine Trajektorie einer Schraube oder eines Drahtes oder einer anderen vordefinierten Form in einer (Bohrer-) Führung relativ zu einer vorgegebenen Achse oder Form ausgerichtet werden muss, die beispielsweise durch einen Knochen in einem registrierten Bild vorgegeben wird. Bei dem Beispiel mit dem Kegel kann der gewünschte Winkel zwischen der Schraubentrajektorie und einer vorgegebenen Achse frei in dem maximal möglichen Bereich von 0° bis 180° eingestellt werden, und diese Einstellung ändert den Öffnungswinkel des Kegels, der an der Spitze der Abbildung des medizinischen bzw. chirurgischen Instruments abgebildet wird. Indem die Kegel-Umfangslinie (Einhüllende) mit der vorgegebenen Knochenoberfläche in Übereinstimmung gebracht wird, wird sichergestellt, dass die Achse des Instruments (Bohrerführung oder Schraubendreher) im gewählten Winkel zu der vorgegebenen Achse oder Oberfläche liegt.

Die Erfindung wird im Weiteren anhand der beiliegenden Zeichnungen und Ausführungsbeispiele näher erläutert. Sie kann alle hierin beschriebenen Merkmale einzeln sowie in jedweder sinnvollen Kombination umfassen. In den Zeichnungen zeigen:
- Figur 1: eine schematische Darstellung eines Navigationssystems und einer erfindungsgemäßen Bildunterstützung;
- Figur 2 und 3: Darstellungen zur Projektion einer Musterform auf eine Bildebene;
- Figur 4: vier verschiedene Darstellungen zur Konusprojektion; und
- Figur 5: eine schematische Darstellung für die virtuelle Einblendung einer Implantatplatte.

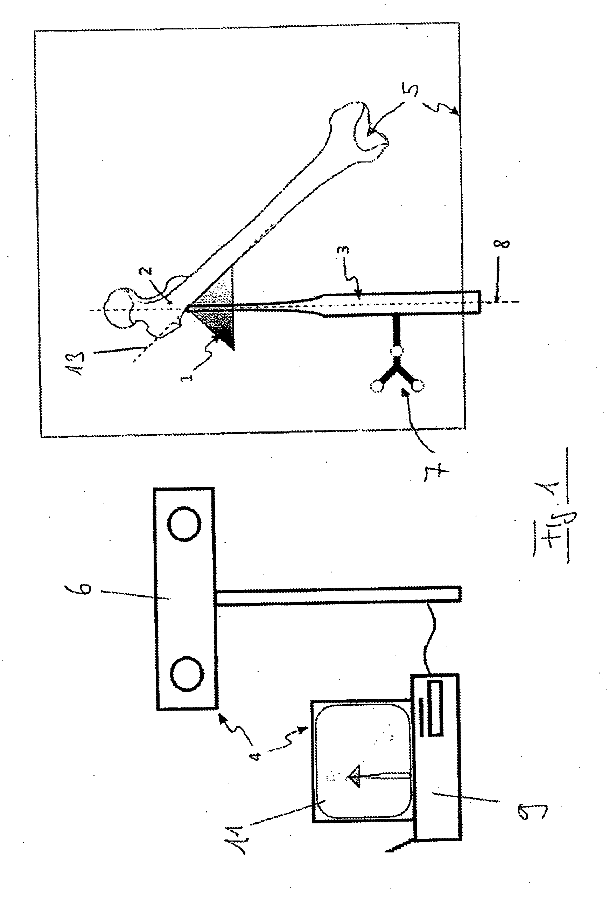

In Figur 1 ist links ein medizintechnisches Tracking- und Navigationssystem dargestellt, das insgesamt das Bezugszeichen 4 trägt. Es besteht aus einer Trackingeinheit 6 mit Kameras, einer Computereinheit 9 und einem Bildschirm 11 (Bildausgabe). Mit der Trackingeinheit 6 wird ein rechts dargestelltes Instrument 3 getrackt, also geortet und verfolgt, und zwar über die Referenzanordnung 7, die am Instrument 3 angebracht ist. Das Instrument hat die Achse 8.

Das Instrument 3 wird getrackt und im richtigen Positionsverhältnis dargestellt, und zwar in Relation zu einem registrierten Patientendatensatz, der beispielsweise durch eine CT- oder ein MR-Scanneraufnahme erhalten wird. Der Bilddatensatz trägt hier das Bezugszeichen 5; er umfasst insbesondere die Bildebene und die Abbildung eines Oberschenkelknochens 2.

An der Spitze des Instruments 3 wird ein Kegel 1 mit einem gewissen Öffnungswinkel in die Bildausgabe eingeblendet, und dieser Kegel 1 bildet hier eine virtuelle Musterform. Wenn der Kegel 1 mit seiner äußeren einhüllenden Geraden an der Ebene liegt, die in Figur 1 mit dem Bezugszeichen 13 gekennzeichnet ist, ist das Instrument richtig ausgerichtet, das heißt die Instrumentenachse 8 zeigt in die richtige Richtung, um beispielsweise Bohrungen durchzuführen oder einen Knochendraht einzusetzen. In der Darstellung wird deutlich, dass die Achse 8 hier genau wie gewünscht durch den Oberschenkelhals hindurchgeht. Der Kegel 1, der bei richtiger Ausrichtung des Instruments an der Linie 13 anliegt, die eine vorgegebene Ebene widerspiegelt, ersetzt also in diesem Fall das Anbringen einer Bohr-Lehre an dem Knochen 3 und vermeidet so die entsprechende invasive Präparation.

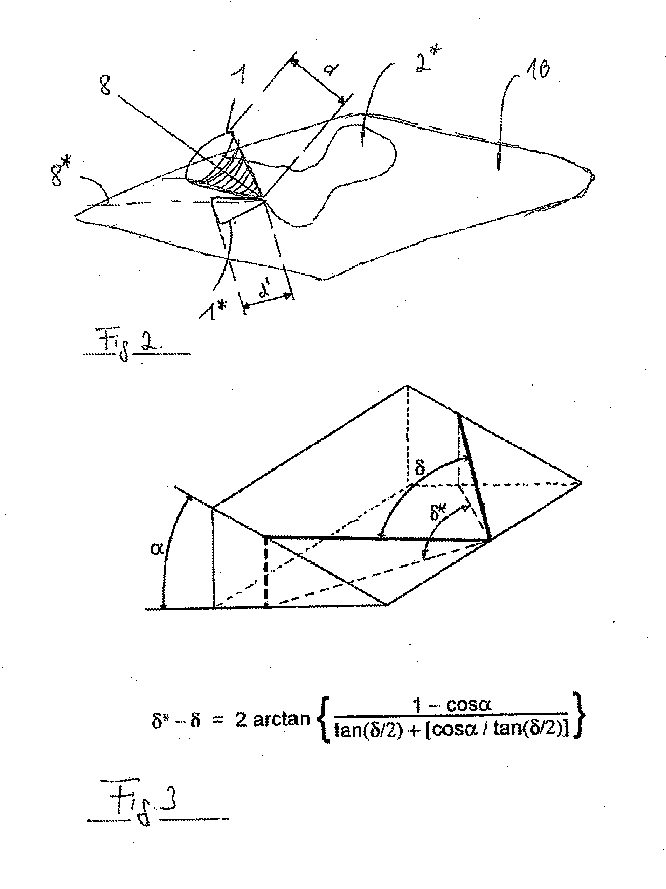

Weil das Instrument separat getrackt wird und in einer gewissen Winkelbeziehung zu der Bildebene stehen wird, in welcher der Patientendatensatz dargestellt wird, muss diese Winkelstellung bei der Darstellung der Musterform, also hier des Kegels 1 in Betracht gezogen werden. Dies geschieht durch Projektion auf die Bildebene, wie in den Figuren 2 und 3 dargestellt. Der Kegel 1 um die Instrumentenachse 8 (jeweils in 3D-Darstellung) wird in der Bildebene 10 abgebildet, in der auch die Knochenabbildung 2* liegt. Dabei entsteht die Abbildung 8* der Achse und 1* des Kegels, der in dieser Abbildung einen anderen Öffnungswinkel haben wird als vorab im Navigationssystem eingegeben und vorbestimmt. Auch die Kegellänge wird in der Projektion verkürzt (d' < d). Wegen der Projektion lässt sich aber die Ansetzung des Kegels an die Hilfslinie (Kegeleinhüllende an der Linie 13 in Figur 1) wieder richtig durchführen und optisch überwachen. Die Figur 3 zeigt, wie eine Winkeländerung δ* - δ bei einer Verkippung um den Winkel α dann vorkommt, wobei die Winkeländerung dann den veränderten projizierten Winkel widerspiegelt. Die Figur 3 gibt ferner noch die mathematische Beziehung für die Winkeländerung an, welche bei der Projektion im Bildmaterial durch das Navigationssystem verwendet werden kann.

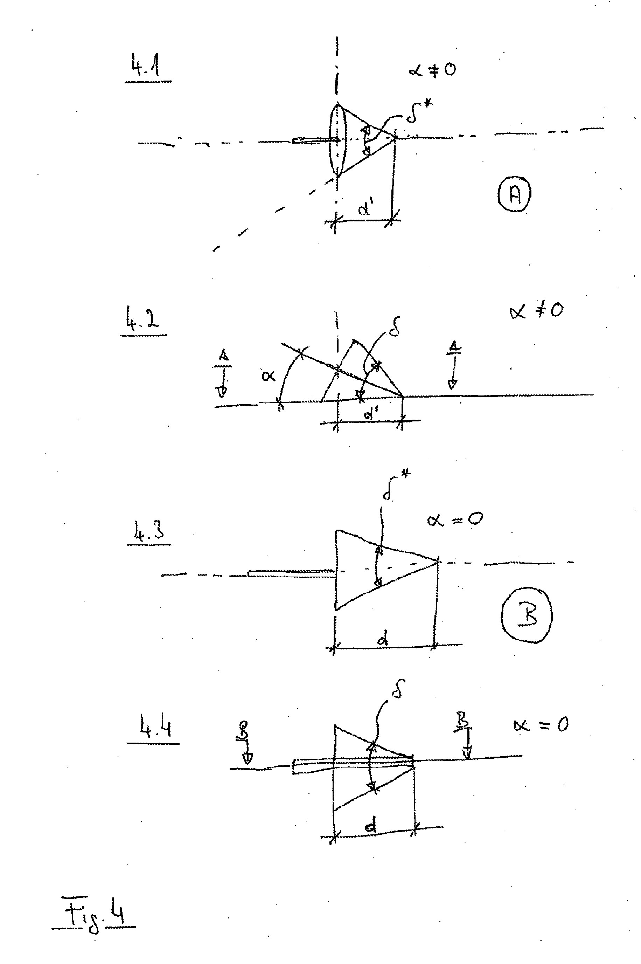

Die Figur 4 zeigt in einzelnen Darstellungen nochmals etwas genauer die Abbildungsverhältnisse für gegenüber der Bildebene geneigte Instrumente (4.1 und 4.2) sowie für ein Instrument in der Bildebene (4.3 und 4.4). Schematisch ist ein gegenüber der Bildebene geneigt angeordneter Konus in den beiden Einzeldarstellungen 4.1 und 4.2 gezeigt, wobei die Einzeldarstellung 4.1 einen Blick von oben auf die Bildebene A und die Einzeldarstellung 4.2 einen Blick parallel zur Bildebene A zeigen. Man sieht, dass in der Ansicht 4.1 der abgebildete Konuswinkel (Kegelwinkel) δ* etwas kleiner ist als der tatsächliche Konusöffnungswinkel δ, der in der Darstellung 4.2 unverzerrt sichtbar wird. Die Kegelabbildung wird auch insgesamt kürzer, sie nimmt nämlich bei der Abbildung eines schräg gehaltenen Instruments (α ≠ 0) die Länge d' an. Dies ergibt sich insbesondere durch einen Vergleich mit den Darstellungen 4.3 und 4.4, welche den Fall zeigen, bei dem die Achse des Instruments und somit auch des Kegels in der Bildebene B liegt. Damit wird der Elevationswinkel α gegen die Bildebene null, und es entstehen keine Verzerrungen bei der Projektion, das heißt der Öffnungswinkel δ entspricht dem projizierten Öffnungswinkel δ*, und auch die Konuslänge d wird korrekt abgebildet; d' ist also für denselben Konus kleiner als d. Auch für die Darstellung 4.3 und 4.4 gilt, dass in 4.3 ein Blick von oben auf die Ebene B und in 4.4 ein Blick parallel zur Ebene B aufgezeigt wird.

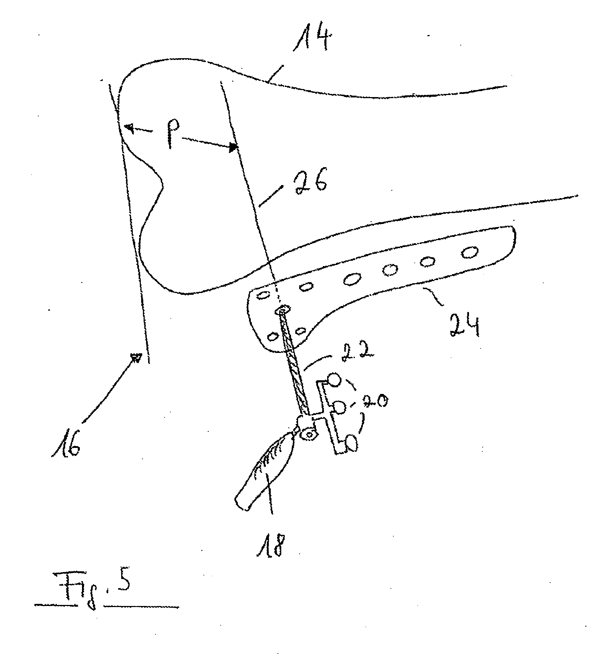

Die Figur 5 soll noch aufzeigen, dass nicht lediglich geometrische Elemente wie zum Beispiel der Kegel 1 als Musterform virtuell dargestellt werden können. Vielmehr eignen sich auch virtuelle Abbildungen von Operationshilfsmitteln, zum Beispiel Implantaten, Schrauben, etc. zur virtuellen Darstellung bei der Navigationsunterstützung. In Figur 5 ist eine solche Platte 24 dargestellt. Vordigitalisierte Versionen solcher Platten oder anderer chirurgischer Hilfsmittel stehen oft schon bereit, weil diese oft in einheitlicher Größe oder in verschiedenen Größen schon zur Verfügung gestellt werden und als Templates navigationsfähig gemacht werden. Man kann also beispielsweise das Instrument 18, hier eine Bohrer-Führung mit der Markeranordnung 20 tracken, so dass die Achse 26 der Hülse 22 als Einblendung auf dem Patientendatensatz gezeigt werden kann, der hier einen Knochenkopf 14 (Femur distal) darstellt. Als Musterform wird dann das Template 24 für eine Platte zusätzlich eingeblendet, das durch ein bestimmtes Loch in einem bestimmten Winkel festgebohrt werden soll. Die Anordnung der Platte lässt sich durch die Anordnung des Lochs gegenüber der Hülse (Bohrerführung) 22 bestimmen, und die Bohrrichtung durch die parallele Anordnung der Achse 26 zu der distalen Kniegelenksachse, die hier schematisch mit 16 angedeutet ist. Die beiden Hilfslinien 16 und 26 (Achse) verlaufen parallel, was durch den Buchstaben P angedeutet werden soll.

Damit lässt sich also als virtuelle Musterform auch zum Beispiel eine Platte 24 navigieren, und die Ausrichtung des Instruments 18 mit der Bohrerhülse 22 (Bohrerführung) kann bildlich unterstützt geplant werden.

## Patentansprüche

1. Verfahren zur bildlichen Unterstützung der Ausrichtung eines medizinischen Instruments (3), bei dem mittels eines medizintechnischen Navigationssystems (4) das Instrument (3) bildlich auf einer Bildausgabe (11) in einer positionellen Relation zu einer Abbildung eines Patientenkörperteils(2) dargestellt wird, wobei an der Abbildung des Instruments (3) oder in bestimmter Positionierung zur Abbildung eines charakteristischen Instrumententeil (8) eine Orientierungshilfe als virtuelle Musterform (1) dargestellt wird, und wobei die Musterform eine an der Instrumentenachse ausgerichtete Kegelform (1) ist.

2. Verfahren nach Anspruch 1, bei dem spezielle geometrische Eigenschaften (13, 16), wie Achsen oder Ebenen oder Landmarken bzw. landmarkenartige Abschnitte, des Patientenkörperteils (2) bildlich als Hilfsabbildungen dargestellt werden, an denen die virtuelle Musterform (1) ausgerichtet werden kann bzw. wird.

3. Verfahren nach Anspruch 1 oder 2, bei dem die Musterform an das Patientenkörperteil oder einen charakteristischen Abschnitt davon angepasst ist.

4. Verfahren nach einem der Ansprüche 1 bis 3, bei dem die Musterform durch Einstellungen am Navigationssystem veränderbar, anpassbar oder austauschbar bzw. aus einer Anzahl von Mustern auswählbar ist.

5. Verfahren nach einem der Ansprüche 1 bis 4, bei dem die Musterform eine Winkelorientierung des Instruments zum Patientenkörperteil unterstützt, insbesondere einen Winkelbereich um eine Achse (8) des Instruments (3) darstellt.

6. Verfahren nach Anspruch 5, bei dem die Kegelspitze an der Instrumentenspitze ausgerichtet ist.

7. Verfahren nach einem der Ansprüche 1 bis 6, bei dem die Musterform als Projektion in die Bildausgabe eingefügt wird.

8. Verfahren nach Anspruch 7, bei dem die Musterform angepasst auf die Bildebene der Patientenkörperteil-Darstellung projiziert und in der Projektion bildlich dargestellt wird.

9. Verfahren zur Planung einer optimierten Instrumentenausrichtung mit Hilfe eines Bildunterstützungsverfahrens gemäß einem der Ansprüche 1 bis 8.

## Claims

1. A method for visually assisting the alignment of a medical instrument (3), wherein the instrument (3) is visually displayed on an image output (11) in a positional relationship to a representation of a part (2) of a patient's body by means of a medical navigation system (4), wherein an orientation aid is displayed as a virtual model shape (1) at the representation of the instrument (3) or in a particular positioning with respect to the representation of a characteristic part (8) of the instrument, and wherein the model shape is a conical shape (1) which is aligned with respect to the axis of the instrument.

2. The method according to claim 1, wherein specific geometric characteristics (13, 16) such as axes or planes or landmarks and/or landmark-like portions of the part (2) of the patient's body are visually displayed as auxiliary representations, with respect to which the virtual model shape (1) can be and/or is aligned.

3. The method according to claim 1 or 2, wherein the model shape is adapted to the part of the patient's body or to a characteristic portion of it.

4. The method according to any one of claims 1 to 3, wherein the model shape can be altered, adapted or exchanged using settings on the navigation system and/or can be selected from a number of models.

5. The method according to any one of claims 1 to 4, wherein the model shape assists an angular orientation of the instrument with respect to the part of the patient's body, and in particular displays an angular range about an axis (8) of the instrument (3).

6. The method according to claim 5, wherein the tip of the cone is aligned with respect to the tip of the instrument.

7. The method according to any one of claims 1 to 6, wherein the model shape is introduced into the image output as a projection.

8. The method according to claim 7, wherein the model shape is projected, in an adapted form, onto the image plane of the representation of the part of the patient's body and visually displayed in the projection.

9. A method for planning an optimised instrument alignment with the aid of an image assisting method in accordance with any one of claims 1 to 8.

## Revendications

1. Procédé pour assister visuellement l'alignement d'un instrument médical (3), dans lequel l'instrument (3) est représenté visuellement sur une sortie d'image (11) dans une relation positionnelle par rapport à une image d'une partie corporelle d'un patient (2) au moyen d'un système de navigation médical (4), dans lequel une aide à l'orientation est représentée sous une forme de modèle virtuelle (1) sur l'image de l'instrument (3) ou dans un positionnement particulier par rapport à l'image d'une partie d'instrument caractéristique (8), et dans lequel la forme de modèle est une forme conique (1) alignée sur l'axe de l'instrument.

2. Procédé selon la revendication 1, dans lequel des caractéristiques géométriques spéciales (13, 16), telles que des axes ou des plans ou des points de repère ou des portions analogues à des points de repère, de la partie corporelle du patient (2) sont représentés visuellement sous forme d'images auxiliaires sur lesquelles la forme de modèle virtuelle (1) peut être ou est alignée.

3. Procédé selon la revendication 1 ou 2, dans lequel la forme de modèle est adaptée à la partie corporelle du patient ou à une portion caractéristique de celle-ci.

4. Procédé selon l'une quelconque des revendications 1 à 3, dans lequel la forme de modèle peut être modifiée, adaptée ou échangée en utilisant des réglages sur le système de navigation, ou peut être choisie parmi un ensemble de modèles.

5. Procédé selon l'une quelconque des revendications 1 à 4, dans lequel la forme de modèle aide à une orientation angulaire de l'instrument par rapport à la partie corporelle du patient, en particulier représente une plage angulaire autour d'un axe (8) de l'instrument (3).

6. Procédé selon la revendication 5, dans lequel le sommet du cône est aligné sur la pointe de l'instrument.

7. Procédé selon l'une quelconque des revendications 1 à 6, dans lequel la forme de modèle est introduite dans la sortie d'image sous la forme d'une projection.

8. Procédé selon la revendication 7, dans lequel la forme de modèle est projetée de manière adaptée sur le plan d'image de la représentation de la partie corporelle du patient et est représentée visuellement dans la projection.

9. Procédé pour planifier un alignement d'instrument optimisé à l'aide d'un procédé d'assistance visuelle selon l'une quelconque des revendications 1 à 8.
